# EUROPEAN PATENT SPECIFICATION

(11) **EP 1 714 626 B1**
(45) Date of publication and mention of the grant of the patent: **24.08.2016**
(21) Application number: 05075921.6
(22) Date of filing: 19.04.2005
(51) Int. Cl.: A61F 5/44, F16K 5/02

(54) **A Tap for drainage of a bag, such as a urine collection bag or the like and a method of manufacturing such tap**
Hahn für die Drainage eines Beutels, insbesondere eines Urinbeutels und Verfahren zu seiner Herstellung
Robinet de drainage d'un sac, en particulier d'un sac collecteur d'urine et procédé pour sa fabrication

(43) Date of publication of application: 25.10.2006
(73) Proprietor: Unomedical A/S, 3460 Birkerød (DK)
(72) Inventor: Hansen, Trygve Kalf, 4040 Jyllinge (DK); Einarsson, Per, 4600 Koge (DK)
(74) Representative: Hoffmann Dragsted A/S

(56) References cited:
- WO-A-00/59416
- GB-A- 2 129 912
- GB-A- 2 166 222
- GB-A- 2 236 575
- US-A- 4 147 184

## Description

The present invention relates to a tap for drainage of a urine collection bag comprising a valve housing having a first and second flow passage and a substantially cylindrical inner space, a valve member with a rotatable valve element and operation means for rotating the valve element in the housing between a closed valve position and an open valve position, said valve member having a means for snap-engagement for mounting onto an annular rim flange groove on the housing. The invention also relates to a method of manufacturing a valve member for such tap.

When a urine bag is attached to the leg for daytime use, it is usual to provide a closure valve functioning as a tap for draining the urine bag by activating the valve tap.

A tap of this kind is known from GB-A-2 166 222. This tap discloses a valve member with a lever arm integrally from thereon. This valve member is snap-mounted to the valve housing and rotatable between a closed position, where the arm is parallel to the flow passages of the valve housing and an open position, where the arm is rotated to a position orthogonal to the flow passages as the arm is moved towards a stop on the outside of the housing.

A similar tap of this kind is known from e.g. WO-A-00/59416 US patent no. 6,722,624 or WO-A-91/03217. In this tap, a relative angular motion between the valve member and the arm is provided so that the arm is moved between two parallel positions, i.e. an angular movement of 180°, for rotating the valve member 90°, for opening and closing the valve. Hereby, the lever arm need not project unduly in either open or closed positions of the tap.

By providing a tap with the lever arm in a parallel position in both valve positions, the risk of accidental operation of the valve is somewhat reduced. However, there is still a substantial risk that the tap lever arm is inadvertently moved away from its open or closed position. The valve may be even just slightly opened if the lever arm is unintentionally moved as a result of the person's activities during the day, or at night, as the lever arm may be pressed against the person's body when the person turns over in his sleep. In order to obtain this function, the lever must be mounted directly to the housing in a manner which allows it to rotate on the housing as it engages the valve member for opening and closing the flow through the tap.

As snap-engagement means includes an annular rim portion with an inwardly projecting flange on the valve member which snaps into an annular groove on the outside of the housing. This "undercut" demands a somewhat complicated moulding technique, as it requires a collapsible mould core. This mould core extends from below to form the cavity for the collar and the annular, inwardly protruding flange. To deform, the mould core must be collapsed, which results in a slow moulding process and extraordinary expensive moulding tools.

Therefore, it is an object of the invention to provide a tap of the initially mentioned kind and a manufacturing method therefore which simplifies the moulding process and provides a speedier and less expensive manufacturing method.

This object is achieved by a tap of the afore-mentioned kind, wherein the means for snap-engagement comprises an annular collar with at least two retention flange sections with inwardly projecting engagement hooks for cooperating with the rim flange groove for rotatably mounting the valve member onto the housing, and that said at least two rim flange sections are annularly spaced apart by open non-engaging sections.

Hereby, a tap is provided which is inexpensive in its manufacture, as the valve member is simple to produce despite the required mounting flanges. In the preferred embodiment of a valve member of a tap according to the invention, three retention flange sections are provided equally spaced on the collar.

In a first embodiment of a tap according to the invention, the operation means of the valve member is a lever integrally formed with rotatable valve element. Hereby, a tap is provided with is particular simple to operate and inexpensive to produce since it consists of only two parts.

In a second embodiment of a tap according to the invention, the operation means of the valve member is a lever engaging rotatable valve element and that the engagement means are formed on the lever. Hereby, a more advanced tap may be provided where the operating lever may be turned between two positions both generally parallel to the flow path through the tap whereas the valve member is only turned approx. 90° between an open and a closed position.

A method of manufacturing a valve member for a tap including the steps of providing a mould defining an inner moulding space, providing a plurality of mould cores for forming at least two retention flange sections with axially inwardly projecting engagement hooks and cooperating with the external forming parts of the mould for forming an annular collar from which the inward projecting hooks are extending, and that said mould cores are inserted into the mould from the side of the mould that the resulting valve member of the moulding process is ejected from the mould, moulding the valve member by forwarding moulding material into the mould after the mould cores are inserted into the mould form, and deforming the moulded valve member by removing the engagement hook forming mould cores in one direction, and ejecting the valve member in substantially the same direction.

By a tap according to the invention and by a method of manufacturing a valve member for this tap, an injection moulding tool of a relative simple kind may be provided. In order to form the snap-engagement flanges, no collapsible mould cores are required. This results in a relative inexpensive moulding tool which in turn results in a cost-effective manufacture of the tap.

In the following, the invention is described in more detail under reference to the accompanying drawings, in which:
- Fig. 1: shows a cross-section of a tap according to the invention;
- Fig. 2: is a top view of the valve housing of a tap according to the invention;
- Fig. 3: is a bottom view of the valve member of a tap according to the invention;
- Fig. 4: shows a tap according to the invention in an open position;
- Fig. 5: shows the tap in a closed position;
- Figures 6 and 7: are perspective views of the valve plug member of a tap according to the invention;
- Figures 8 and 9: are schematic views of manufacturing techniques for moulding the valve member; and
- Figures 10 and 11: are schematic views of the moulding process for moulding a valve member for a tap according to the invention.

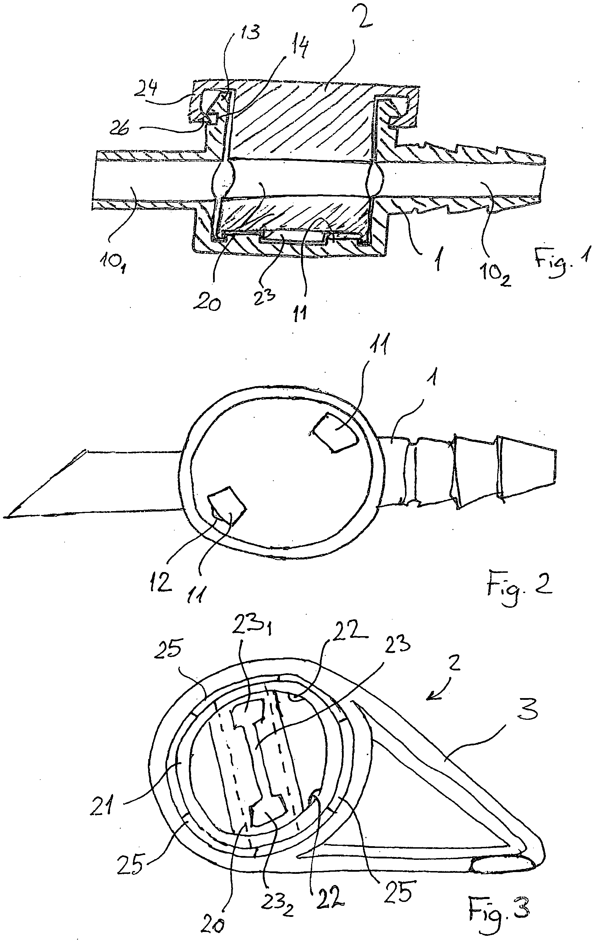

As shown in the figures 1 to 3, the tap for a urine collection bag includes a valve housing 1 with a flow inlet 10₂ and outlet 10₁ cooperating with a valve member 2, which is rotatable in the housing 1 so that a flow passage 20 is aligned with the flow inlet 10₂ and outlet 10₁ of the housing 1 in an open position and where the flow passage 10 is substantially 90° off-set from the housing flow passages 10 in a closed position. The valve member 2 includes an arm 3 for rotating the valve plug member 2 between the open and closed positions.

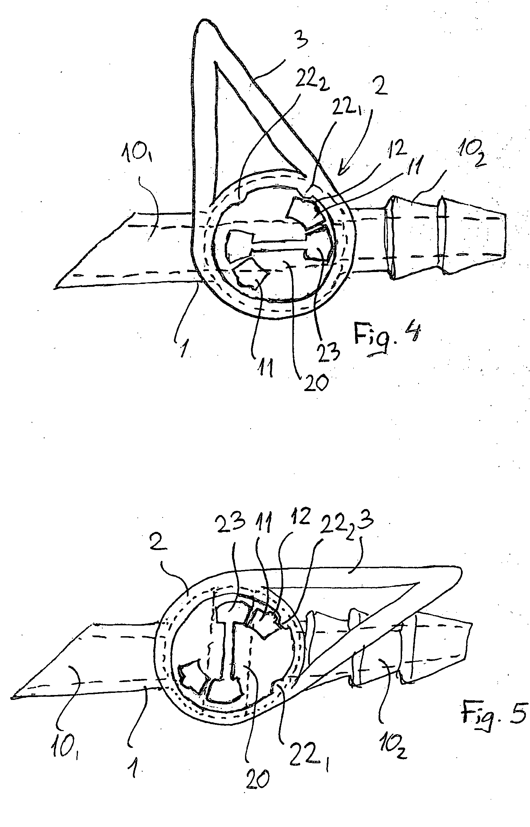

To control the angular movement of the valve member 2 in the valve housing 1, the inner space of the valve housing 1 is cylindrical and the valve plug member 2 is formed correspondingly. Two upwardly protruding stops 11 are formed in the bottom of the valve housing 1 at an angular distance of approx. 180° from each other and with a certain distance from the periphery of the circular bottom surface of the housing. At least one of the stops 11 is provided with a small notch 12, which is provided on the radial outermost side of the stop 11. The valve plug 2 is provided with a cylindrical body 21 with a wall thickness which is smaller than the distance from the notch 12 to the cylindrical inner sidewall of the housing 1 so that the valve member body 21 is allowed to pass the notch 12. The inner side of the cylindrical body 21 is provided with two, radially inwardly facing notches 22 in the end section of the cylindrical body 21. The end section is also provided with a stop member 23 including two stop heads 23₁, 23₂ radially spaced apart at 180°, which are provided for engagement with the stops 11 of the valve housing 1. The two notches 22 are placed with an angular displacement of less than 90°, e.g. approximately 80°, so that when the valve member 2 is rotated near to one stop 11, the notch 12 pass a first notch 22₁ just before the stop member 23 abuts the stop 11, whereby the valve member 2 is retained in this stop position. Similarly, the valve plug member 2 may be retained in a second stop position at the second notch 22₂. The direction of the flow passage 20 of the valve plug member 2 is coordinated with the positions of the stops 23 and the notches 22, so that the first stop is an open position, where the flow passage 20 is aligned with the flow system 10 of the housing 1 (see fig. 4), and the second position is a closed position, where the flow passage 20 is generally orthogonal to the flow system 10 of the housing 1 (see fig 5).

By the cooperating stops 11, 23 and notches 12, 22, the valve plug member 2 is retained in its two operative positions, as shown in figures 4 and 5. This eliminates the risk of leakage and accidental opening of the tap of the urine collection bag.

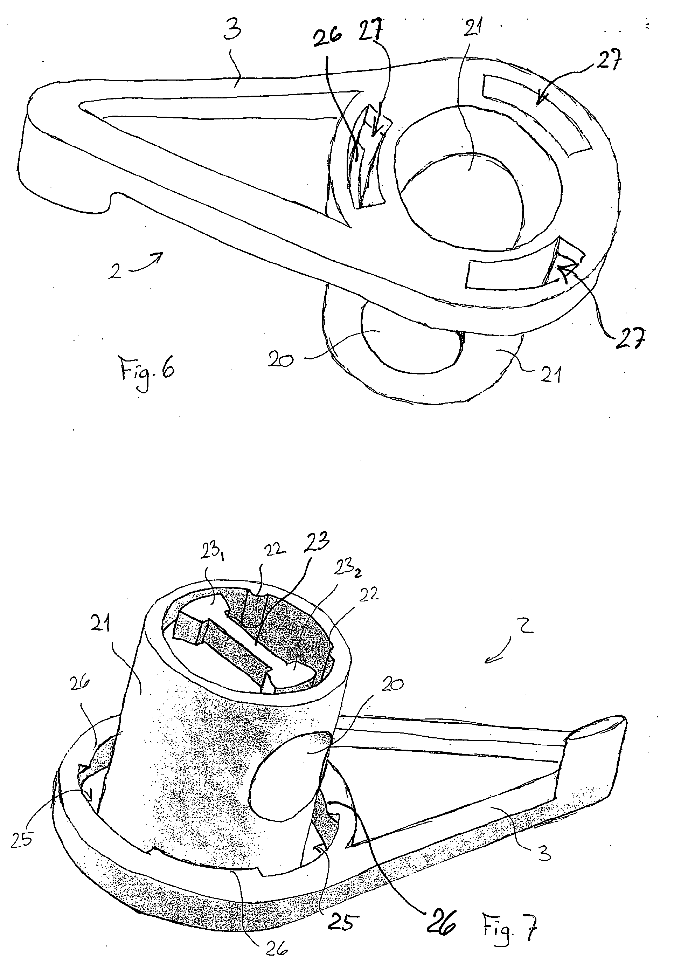

A preferred embodiment of a valve member 2 is shown in figures 6 and 7. An arm 3 for activating the tap is integrally formed in the valve member 2. With reference to fig. 1, the valve member 2 is provided with an interrupted annular collar 24, which cooperates with an annular end section 13 of the housing 1, where the collar 24 has an inwardly protruding portion 26 and is retained in an annular groove 14 of the housing 1 by a snap-engagement. Hereby, the valve member 2 is secured to the housing 1 in a rotatable manner.

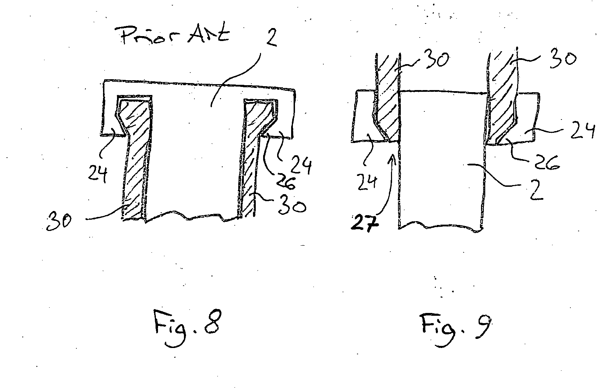

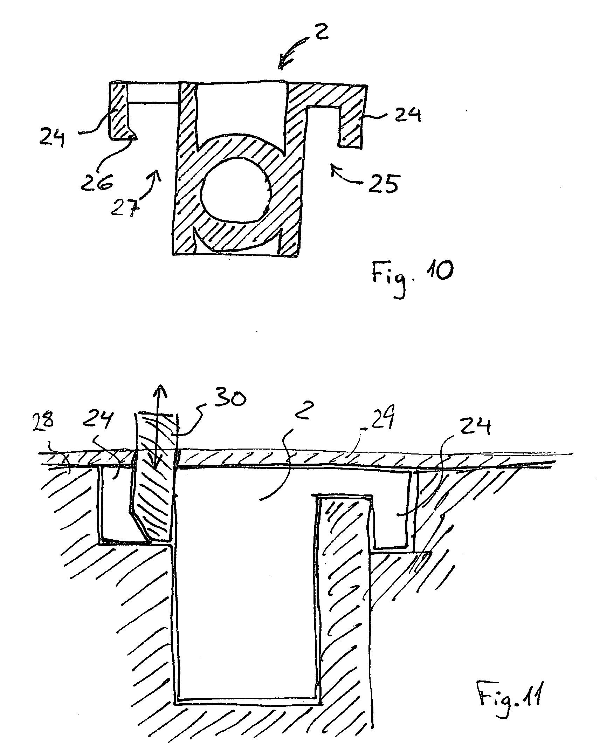

The moulding of the valve member 2 is somewhat difficult, as the moulding of the annular collar 24 in the known snap-engagement tap designs requires a collapsible mould core 30. This mould core 30 extends from below to form the cavity for the collar 24 and the annular, inwardly protruding flange 26 (see fig. 8). To deform, the mould core 30 must be collapsed, which results in a slow moulding process and extraordinary expensive moulding tools. Instead, it is realised that a multiple of retention flanges 26 (see figures 6, 7, 9 and 10) may be provided instead of one annular flange 26 as shown in fig. 8. Hereby, open spaces 25, 27 are provided in between the flange portions 26 as shown in fig. 3, 6, 7 and 9. This involves two kinds of open spaces in between each other, a first kind of space 27 between the flange portions 26 and the cylindrical surface of the valve member 2 and a second kind between the collar 24 and the surface of the valve member 2 in the sections where the collar 24 is without a retention flange 26. As shown in fig. 9 and 10, this means that the mould core 30 may extend from above and downwards into the inner mould space of the mould 28, which makes it easy and quicker to deform the moulded valve member from the mould 28.

As shown schematically in fig. 11, the mould comprises external form parts 28, 29 and mould cores 30 (only one is shown in the side cross-section view in fig. 11) for forming the snap-engagement members 26. Other mould cores (not shown) may of cause also be provided to form various other details on the valve member. The melted plastic material is injected into the mould through injection channels (not shown).

The invention is described with reference to a preferred embodiment. However, it is realised that other variants of the invention may be provided without departing from the scope of the invention as defined in the accompanying claims.

## Claims

1. A tap for drainage of a urine collection bag comprising a valve housing (1) having a first and second flow passage (10₁, 10₂) and a substantially cylindrical inner space, a moulded valve member (2) with a rotatable valve element and operation means (3) for rotating the valve element in the housing (1) between a closed valve position and an open valve position, said valve member (2) having a means for snap-engagement for mounting onto an annular rim flange groove (13, 14) on the housing (1), said means for snap-engagement comprising an annular collar (24),
**characterised in that**
the annular collar (24) comprises at least two open retention flange sections (26) with inwardly projecting engagement hooks for cooperating with the rim flange groove (13, 14) for rotatably mounting the valve member onto the housing, and that said at least two open retention flange sections (26) are annularly spaced apart by non-engaging sections (25).

2. A tap according to claim 1, wherein three retention flange sections (26) are provided equally spaced on the collar (24).

3. A tap according to claim 1 or 2, wherein the operation means of the valve member (2) is a lever integrally formed with rotatable valve element.

4. A tap according to claim 1 or 2, wherein the operation means of the valve member (2) is a lever engaging rotatable valve element and that the engagement means are formed on the lever.

5. A method of manufacturing a valve member (2) for a tap according to any of claims 1 to 4, said method including the steps of:
providing a mould (28, 29) defining an inner moulding space,
providing a plurality of mould cores (30) for forming at least two retention flange sections (26) with axially inwardly projecting engagement hooks (26) and cooperating with the external forming parts of the mould (28, 29) for forming an annular collar (24) from which the inward projecting hooks (26) are extending,
inserting said mould cores (30) into the mould from the side of the mould that the resulting valve member of the moulding process is ejected from the mould,
moulding the valve member by forwarding moulding material into the mould after the ; mould cores are inserted into the mould form, and
removing the mould parts forming the moulded valve member including removing the engagement hook forming mould cores in one direction, and
ejecting the valve member in substantially the same direction.

## Patentansprüche

1. Hahn zum Entleeren eines Urin-Sammelbeutels, der ein Ventilgehäuse (1) umfasst, das einen ersten und zweiten Flusskanal (10₁, 10₂) und einen im Wesentlichen zylindrischen Innenraum hat, sowie ein geformtes Ventilteil (2) mit einem drehbaren Ventilelement und einem Betätigungsmittel (3) zum Drehen des Ventilelements im Gehäuse (1) zwischen einer geschlossenen Ventilposition und einer offenen Ventilposition, wobei das Ventilteil (2) ein Mittel für einen Schnappeingriff zur Montage auf einer ringförmigen Kranzflanschnut (13, 14) auf dem Gehäuse (1) hat und wobei das Mittel für den Schnappeingriff einen ringförmigen Kragen (24) umfasst,
**dadurch gekennzeichnet, dass**
der ringförmige Kragen (24) wenigstens zwei Offen-Halteflansch-Abschnitte (26) mit einwärts vorstehenden Eingriffhaken für die Zusammenarbeit mit der Kranzflanschnut (13, 14) umfasst, um das Ventilteil drehbar auf dem Gehäuse anzubringen, und dadurch, dass die wenigstens zwei Offen-Halteflansch-Abschnitte (26) durch nicht eingreifende Abschnitte (25) in Ringrichtung voneinander beabstandet sind.

2. Hahn nach Anspruch 1, wobei drei Halteflansch-Abschnitte (26) äquidistant auf dem Kragen (24) vorgesehen sind.

3. Hahn nach Anspruch 1 oder 2, wobei das Betätigungsmittel des Ventilteils (2) ein Hebel ist, der integral mit dem drehbaren Ventilelement ausgebildet ist.

4. Hahn nach Anspruch 1 oder 2, wobei das Betätigungsmittel des Ventilteils (2) ein Hebel ist, der mit dem drehbaren Ventilelement im Eingriff ist und wobei das Eingriffmittel auf dem Hebel ausgebildet ist.

5. Verfahren zur Herstellung eines Ventilteils (2) für einen Hahn nach einem der Ansprüche 1 bis 4, wobei das Verfahren die folgenden Schritte umfasst:
Bereitstellen einer Form (28, 29), die eine innere Formkammer definiert,
Bereitstellen mehrerer Formkerne (30) zum Formen wenigstens zweier Halteflansch-Abschnitte (26) mit axial einwärts vorstehenden Eingriffhaken (26), wobei sie mit den äußeren Formungsteilen der Form (28, 29) zusammenarbeiten, um einen ringförmigen Kragen (24) zu formen, von dem ausgehend die einwärts vorstehenden Haken (26) sich erstrecken,
Einsetzen der Formkerne (30) in die Form von der Seite der Form, zu der das im Formvorgang erzeugte Ventilteil aus der Form ausgestoßen wird,
Formen des Ventilteils durch das Zuführen von Formmaterial in die Form nachdem die Formkerne in die Form eingesetzt sind, und
Entfernen der Formteile, die das geformte Ventilteil formen, wobei dies das Entfernen der Formkerne zum Formen der Eingriffhaken in eine Richtung einschließt, und
Ausstoßen des Ventilteils in im Wesentlichen derselben Richtung.

## Revendications

1. Robinet pour le drainage d'un sac de collecte d'urine comprenant un logement de valve (1) comportant un premier et un second passages d'écoulement (10₁, 10₂) et un espace intérieur sensiblement cylindrique, un élément de valve (2) moulé avec un élément de valve tournant et des moyens d'actionnement (3) pour faire tourner l'élément de valve dans le logement (1) entre une position de valve fermée et une position de valve ouverte, ledit élément de valve (2) comportant des moyens pour prise par emboitement pour montage sur une rainure de rebord de jante (13, 14) annulaire sur le logement (1), lesdits moyens pour prise par emboitement comprenant un collier annulaire (24),
**caractérisé en ce que**
le collier annulaire (24) comprend au moins deux sections de rebord de retenue ouvertes (26) avec des crochets de prise faisant saillie vers l'intérieur pour coopérer avec la rainure de rebord de jante (13, 14) pour montage à rotation de l'élément de valve sur le logement, et **en ce que** lesdites au moins deux sections de rebord de retenue ouvertes (26) sont espacées annulairement par des sections non en prise (25).

2. Robinet selon la revendication 1, dans lequel trois sections de rebord de retenue (26) sont fournies également espacées sur le collier (24).

3. Robinet selon la revendication 1 ou 2, dans lequel les moyens d'actionnement de l'élément de valve (2) sont un levier formé d'un seul bloc avec l'élément de valve tournant.

4. Robinet selon la revendication 1 ou 2, dans lequel les moyens d'actionnement de l'élément de valve (2) sont un levier venant en prise avec l'élément de valve tournant et que les moyens de prise sont formés sur le levier.

5. Procédé de fabrication d'un élément de valve (2) pour un robinet selon l'une quelconque des revendications 1 à 4, ledit procédé incluant les étapes consistant à :
fournir un moule (28, 29) définissant un espace de moulage intérieur,
fournir une pluralité de noyaux de moule (30) pour former au moins deux sections de rebord de retenue (26) avec des crochets de prise faisant saillie vers l'intérieur (26) et coopérant avec les parties extérieures formant le moule (28, 29) pour former un collier annulaire (24) depuis lequel les crochets faisant saillie vers l'intérieur (26) s'étendent,
insérer lesdits noyaux de moule (30) dans le moule depuis le côté du moule que l'élément de valve résultant du processus de moulage est éjecté du moule,
mouler l'élément de valve en avançant un matériau de moulage dans le moule après que les noyaux de moule sont insérés dans la forme de moule, et
enlever les parties de moule formant l'élément de valve moulé incluant d'enlever les noyaux de moule formant les crochets de prise dans une direction, et
éjecter l'élément de valve dans sensiblement la même direction.
